# EUROPÄISCHE PATENTSCHRIFT

(11) **EP 1 054 573 B1**
(45) Veröffentlichungstag und Bekanntmachung des Hinweises auf die Patenterteilung: **28.02.2007**
(21) Anmeldenummer: 00105203.4
(22) Anmeldetag: 13.03.2000
(51) Int. Cl.: H04R 25/00, A61F 2/18

(54) **Vorrichtung zum mechanischen Ankoppeln eines in einer Mastoidhöhle implantierbaren elektromechanischen Hörgerätewandlers**
Device for mechanical coupling of a electromechanical hearing aid transducer implantable in a cavity in the mastoid
Dispositif pour le couplage mécanique d'un transducteur électromécanique de prothèse auditive implantable dans une cavité dans la mastoidite

(30) Priorität: 21.05.1999 DE 19923403
(43) Veröffentlichungstag der Anmeldung: 22.11.2000
(73) Patentinhaber: PHONAK AG, 8712 Stäfa (CH)
(72) Erfinder: Leysieffer, Hans, Dr.-Ing. (Dipl.-Ing.), 82024 Taufkirchen (DE); Müller, Gerd M., Dr. rer. nat. (Dr. Dipl.-Phys.), 85716 Lohhof (DE)
(74) Vertreter: Schwan, Gerhard

(56) Entgegenhaltungen:
- EP-A- 0 901 779
- WO-A-92/09181

## Beschreibung

Die Erfindung betrifft eine Vorrichtung zum mechanischen Ankoppeln eines zu mechanischen Schwingungen anregbaren ausgangsseitigen Wandlerteils eines außerhalb des Mittelohrbereiches in einer artifiziellen Mastoidhöhle implantierbaren elektromechanischen Hörgerätewandlers an eine vorgewählte Ankoppelstelle an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran, mit einer biokompatiblen, mechanisch passiven Koppelanordnung, die mit dem ausgangsseitigen Wandlerteil verbunden ist und die im implantierten Zustand von der Mastoidhöhle in die Paukenhöhle reicht sowie mit einem von dem Hörgerätewandler abliegenden Ankoppelende an der Ankoppelstelle anliegt.

Elektronische Maßnahmen zur Rehabilitation eines operativ nicht behebbaren Innenohrschadens haben heute einen wichtigen Stellenwert erreicht. Bei totalem Ausfall des Innenohres sind Cochlea Implantate mit direkter elektrischer Reizung des verbleibenden Hörnerven im routinemäßigen klinischen Einsatz. Bei mittleren bis schweren Innenohrschäden kommen derzeit erstmals volldigitale Hörgeräte zur Anwendung, die eine neue Welt der elektronischen Audiosignalverarbeitung eröffnen und erweiterte Möglichkeiten der gezielten audiologischen Feinanpassung der Hörgeräte an den individuellen Innenohrschaden bieten. Trotz dieser in den letzten Jahren erreichten, erheblichen Verbesserungen der apparativen Hörgeräteversorgung bleiben bei konventionellen Hörgeräten grundsätzliche Nachteile bestehen, die durch das Prinzip der akustischen Verstärkung bedingt sind, das heißt insbesondere durch die Rückwandlung des elektronisch verstärkten Signals in Luftschall. Zu diesen Nachteilen zählen Aspekte wie die Sichtbarkeit der Hörgeräte, mangelnde Klangqualität aufgrund der elektromagnetischen Wandler (Lautsprecher), verschlossener äußerer Gehörgang sowie Rückkoplungseffekte bei hoher akustischer Verstärkung.

Aufgrund dieser prinzipiellen Nachteile besteht seit langem der Wunsch, von konventionellen Hörgeräten mit akustischer Anregung des geschädigten Innenohres abzukommen und diese Geräte durch teil- oder vollimplantierbare Hörsysteme mit einer direkten mechanischen Stimulation zu ersetzen. Implantierbare Hörsysteme unterscheiden sich von konventionellen Hörgeräten: zwar wird das Schallsignal mit einem Wandler (Mikrofon) in ein elektrisches Signal umgewandelt und in einer elektronischen Signalverarbeitungsstufe verstärkt; dieses verstärkte elektrische Signal wird jedoch nicht einem elektroakustischen Wandler (Lautsprecher) zugeführt, sondern einem implantierten elektromechanischen Wandler, dessen ausgangsseitige mechanische Schwingungen unmittelbar, also mit direktem mechanischen Kontakt, dem Mittel- beziehungsweise Innenohr zugeführt werden oder mittelbar durch einen Kraftschluß über einen Luftspalt bei zum Beispiel elektromagnetischen Wandlersystemen. Dieses Prinzip gilt unabhängig von einer teilweisen oder vollständigen Implantation aller notwendigen Systemelemente sowie auch unabhängig davon, ob eine reine Innenohrschwerhörigkeit bei vollständig intaktem Mittelohr oder eine kombinierte Schwerhörigkeit (Mittel- und Innenohr geschädigt) rehabilitiert werden soll. Daher sind in der jüngeren wissenschaftlichen Literatur sowie in zahlreichen Patentschriften implantierbare elektromechanische Wandler sowie Verfahren zur direkten Ankopplung der mechanischen Wandlerschwingungen an das intakte Mittelohr beziehungsweise das Innenohr zur Rehabilitation einer reinen Innenohrschwerhörigkeit sowie auch an verbleibende Ossikel des Mittelohres bei artifiziell oder pathologisch verändertem Mittelohr zur Versorgung einer Schalleitungsschwerhörigkeit sowie deren Kombinationen beschrieben worden.

Als elektromechanisches Wandlerverfahren kommen grundsätzlich alle physikalischen Wandlungsprinzipien in Frage wie elektromagnetisch, elektrodynamisch, magnetostriktiv, dielektrisch und piezoelektrisch. Verschiedene Forschungsgruppen haben sich in den letzten Jahren im wesentlichen auf zwei dieser Verfahren konzentriert: elektromagnetisch und piezoelektrisch. Eine Übersicht über diese Wandlervarianten findet sich bei Zenner und Leysieffer (HNO 1997 Vol. 45, 749 - 774).

Beim piezoelektrischen Verfahren ist eine mechanisch direkte Kopplung der ausgangsseitigen Wandlerschwingungen an die Mittelohrossikel oder direkt an das ovale Fenster notwendig. Beim elektromagnetischen Prinzip kann die Kraftkopplung einerseits über einen Luftspalt erfolgen ("kontaklos"), das heißt, nur ein Permanentmagnet wird durch dauerhafte Fixation in direkten mechanischen Kontakt mit einem Mittelohrossikel gebracht. Andererseits besteht die Möglichkeit, den Wandler vollständig in einem Gehäuse zu realisieren (wobei Spule und Magnet mit kleinstmöglichem Luftspalt gekoppelt sind) und die ausgangsseitigen Schwingungen über ein mechanisch steifes Koppelelement mit direktem Kontakt auf die Mittelohrossikel zu übertragen (Leysieffer et al. 1997 (HNO 1997, Vol. 45, pp. 792-800)).

In der Patentliteratur finden sich einige der oben genannten Realisierungsvarianten sowohl elektromagnetischer wie auch piezoelektrischer Hörgerätewandler: US-A-5 707 338 (Adams et al.), WO 98/06235 (Adams et al.), WO 98/06238 (Adams et al.), WO 98/06236 (Kroll et al.), WO 98/06237 (Bushek et al.), US-A-5 554 096 (Ball), US-A-3 712 962 (Epley), US-A-3 870 832 (Fredrickson), US-A-5 277 694 (Leysieffer et al.), EP-A-0 984 663 (Leysieffer), EP-A-0 984 665 (Leysieffer), US-A-5 015 224 (Maniglia), US-A-3 882 285 (Nunley), US-A-4 850 962 (Schaefer).

Das teilimplantierbare, piezoelektrische Hörsystem der japanischen Gruppe um Suzuki und Yanigahara setzt für eine Implantation des Wandlers das Fehlen der Mittelohrossikel und eine freie Paukenhöhle voraus, um das Piezoelement an den Stapes ankoppeln zu können (Yanigahara et al.: Efficacy of the partially implantable middle ear implant in middle and inner ear disorders. Adv. Audiol.., Vol. 4, Karger Basel (1988), pp. 149-159; Suzuki et al.: Implantation of partially implantable middle ear implant and the indication. Adv. Audiol., Vol. 4, Karger Basel (1988), pp. 160-166). Ebenso wird bei dem Verfahren eines implantierbaren Hörsystems für Innenohrschwerhörige nach US-A-4 850 962 (Schaefer) grundsätzlich der Amboß entfernt, um ein piezoelektrisches Wandlerelement an den Stapes ankoppeln zu können. Dies gilt im wesentlichen auch für weitere Entwicklungen, die auf der SCHAEFER-Technologie basieren und in den oben genannten Patentschriften dokumentiert sind (US-A-5 707 338, WO 98/06235, WO 98/06238, WO 98/06236, WO 98/06237).

Der elektromagnetische Wandler nach Ball ("Floating Mass Transducer FMT", US-A-5 624 376, US-A-5 554 096) wird dagegen bei intaktem Mittelohr mit Titanclips direkt an dem langen Fortsatz des Amboß fixiert. Der elektromagnetische Wandler des teilimplantierbaren Systems nach FREDRICKSON (Fredrickson et al.: Ongoing investigations into an implantable elektromagnetic hearing aid for moderate to severe sensorineural hearing loss. Otolaryngologic Clinics Of Nord America, Vol. 28/1 (1995), pp. 107-121) wird bei ebenfalls intakter Ossikelkette des Mittelohres mechanisch direkt an den Amboßkörper gekoppelt. Das Gleiche gilt für die piezoelektrischen und elektromagnetischen Wandler nach LEYSIEFFER (Leysieffer et al.: Ein implantierbarer piezoelektrischer Hörgerätewandler für Innenohrschwerhörige. HNO 1997/45, pp. 792-800, US-A-5 277 694, EP-A-0 984 663, EP-A-0 984 665). Auch bei dem elektromagnetischen Wandlersystem nach MANIGLIA (Maniglia et al.: Contactless semi-implantable electromagnetic middle ear device for the treatment of sensorineural hearing loss, Otolaryngologic Clinics Of Nord America, Vol. 28/1 (1995), pp. 121-141) wird bei intakter Ossikelkette ein Permanentmagnet mechanisch an der Ossikelkette dauerhaft fixiert, der jedoch über eine Luftspaltkopplung von einer Spule mechanisch angetrieben wird.

Bei den beschriebenen Wandler- und Ankopplungsvarianten sind grundsätzlich zwei Implantationsprinzipien zu unterscheiden:
a) Im Falle des einen Prinzips befindet sich der elektromechanische Wandler mit seinem aktiven Wandlerelement selbst im Mittelohrbereich in der Paukenhöhle, und der Wandler ist dort mit einem Ossikel oder dem Innenohr direkt verbunden (US-A-4 850 962, US-A-5 015 225, US-A-5 707 338, WO 98/06235, WO 98/06238, WO 98/06236, WO 98/06237, US-A-5 624 376, US-A-5 554 096).
b) Bei dem anderen Prinzip befindet sich der elektromechanische Wandler mit seinem aktiven Wandlerelement außerhalb des Mittelohrbereiches in einer artifiziell geschaffenen Mastoidhöhle. Die ausgangsseitigen mechanischen Schwingungen werden dann mittels mechanisch passiver Koppelelemente über geeignete operative Zugänge (natürlicher aditus ad antrum, Eröffnung des chorda-facialis-Winkels oder über eine artifizielle Bohrung vom Mastoid aus) zum Mittel- oder Innenohr übertragen (Fredrickson et al.: Ongoing investigations into an implantable elektromagnetic hearing aid for moderate to severe sensorineural hearing loss. Otolaryngologic Clinics Of Nord America, Vol. 28/1 (1995), pp. 107-121; DE-B-41 04 358.8; EP-A-0 984 663; EP-A-0 984 665).

Bei den Varianten nach a) kann der Wandler als sogenannter "Floating Mass"-Wandler ausgeführt sein, das heißt, das Wandlerelement benötig keine "Reaktio" über eine feste Verschraubung mit dem Schädelknochen, sondern es schwingt aufgrund von Massenträgheitsgesetzen mit seinem Wandlergehäuse und überträgt diese direkt auf ein Mittelohrossikel (US-A-5 624 376, US-A-5 554 096, US-A-5 707 338, WO 98/06236). Dies bedeutet einerseits, daß vorteilhaft auf ein implantierbares Fixationssystem an der Schädelkalotte verzichtet werden kann; andererseits bedeutet diese Variante nachteilig, daß voluminöse artifizielle Elemente in die Paukenhöhle eingebracht werden müssen und deren Langzeit- und Biostabilität insbesondere bei temporären pathologischen Veränderungen des Mittelohres (z.B. otitis media) heute nicht bekannt beziehungsweise gewährleistet sind. Ein weiterer wesentlicher Nachteil besteht darin, daß die Wandler vom Mastoid aus mit ihrer elektrischen Zuleitung ins Mittelohr gebracht und dort mit Hilfe geeigneter operativer Werkzeuge fixiert werden müssen; dies erfordert einen erweiterten Zugang durch den chorda-facialis-Winkel und bringt somit eine latente Gefährdung des in unmittelbarer Nachbarschaft gelegenen Gesichtsnerven (nervus facialis) mit sich.

Bei den Wandlervarianten nach b) muß das Wandlergehäuse mit implantierbaren Positionier- und Fixationssystemen an der Schädelkalotte befestigt werden (vorteilhafte Ausführung DE-A-196 18 964 entsprechend US-A-5 788 711). Ein Nachteil der Varianten nach b) besteht darin, daß eine Vertiefung in die Zielossikel, vorzugsweise mittels geeigneter Laser, eingebracht werden muß, um das Koppelelement applizieren zu können. Dies ist einerseits technisch aufwendig und teuer und bringt andererseits Risiken für den Patienten mit sich. Sowohl bei dem teilimplantierbaren System nach FREDRICKSON (Ongoing investigations into an implantable elektromagnetic hearing aid for moderate to severe sensorineural hearing loss. Otolaryngologic Clinics Of Nord America, Vol. 28/1 (1995), pp. 107-121) wie auch bei dem vollimplantierbaren Hörsystem nach LEYSIEFFER und ZENNER (HNO 1998, Vol. 46, 853-863 und 844-852) wird bei der Ankopplung des schwingenden Wandlerteils an den Amboßkörper zur dauerhaften und mechanisch sicheren Schwingungsübertragung davon ausgegangen, daß die Spitze der Koppelstange, die in die laserinduzierte Vertiefung des Mittelohrossikels eingebracht wird, langfristig eine Osseointegration erfährt, das heißt, die Koppelstange verwächst fest mit dem Ossikel und gewährleistet so eine sichere Übertragung dynamischer Druck- und Zugkräfte. Dieser Langzeiteffekt ist zur Zeit jedoch noch nicht wissenschaftlich nachgewiesen beziehungsweise gesichert. Weiterhin besteht bei dieser Ankopplungsart bei einem technischen Wandlerdefekt der Nachteil, daß eine Entkopplung vom Ossikel zur Entfernung des Wandlers nur mit mechanisch basierten operativen Methoden vorgenommen werden kann, was eine erhebliche Gefährdung des Mittelohres und insbesondere des Innenohres bedeuten kann.

Der wesentliche Vorteil dieser Wandlerausführungsformen nach b) besteht jedoch darin, daß das Mittelohr weitgehend frei bleibt und der Koppelzugang zum Mittelohr ohne größeres Gefährdungspotential des nervus facialis erfolgen kann. Ein vorzugsweises operatives Verfahren hierzu ist in der US-Patentanmeldung 09/168.079 beschrieben. Grundlegende vorteilhafte Formen passiver Koppelelemente zur Übertragung der ausgangsseitigen Wandlerschwingungen vom Mastoid aus zum Mittel- beziehungsweise Innenohr sind in EP-A-0 499 940 (entsprechend US-A-5 277 964), in EP-A-0 901 779 (entsprechend US 09/042.805) und in HNO 1998 Vol. 46, 27 - 37 - Lehner et al.: "Kaltfließende Elemente zur Ankopplung eines implantierbaren Hörgerätewandlers an Gehörknöchelchen oder Perilymphe" beschrieben. Dabei handelt es sich insbesondere um Koppelelemente aus Gold, vorzugsweise weichgeglühtem Feingold, in Form eines C-Bandes für den langen Amboßfortsatz, einer Bandschlaufe für den langen Amboßfortsatz und eines Glöckchens für das Steigbügelköpfchen, wobei sich diese Koppelelemente unter Verwendung von ohrchirurgischen Standardinstrumenten ankoppeln und erforderlichenfalls auch wieder lösen lassen.

Der vorliegenden Erfindung liegt die Aufgabe zugrunde, eine Vorrichtung zum Ankoppeln eines Hörgerätewandlers und zur Übertragung der ausgangsseitigen Wandlerschwingungen an das Mittel- beziehungsweise Innenohr zu schaffen, die unter Beibehaltung der genannten Vorteile der Variante b) einfacher und sicherer applizierbar ist, die während der Implantation im Innenohr notwendige, risikobehaftete Arbeiten minimiert und auch ein unter Umständen später notwendig werdendes Entkoppeln erleichtert sowie eine optimale Schwingungsform der Steigbügelfußplatte begünstigt.

Ausgehend von einer Vorrichtung der aus EP-A-0 901 779 und HNO 1998 Vol. 46, 27-37 bekannten Art, das heißt einer Vorrichtung zum mechanischen Ankoppeln eines zu mechanischen Schwingungen anregbaren ausgangsseitigen Wandlerteils eines außerhalb des Mittelohrbereiches in einer artifiziellen Mastoidhöhle implantierbaren elektromechanischen Hörgerätewandlers an eine vorgewählte Ankoppelstelle an der Ossikelkette, der Steigbügelfußplatte oder einer das runde Fenster oder ein artifizielles Fenster abschließenden Membran, mit einer biokompatiblen, mechanisch passiven Koppelanordnung, die mit dem ausgangsseitigen Wandlerteil verbunden ist und die im implantierten Zustand von der Mastoidhöhle in die Paukenhöhle reicht sowie mit einem von dem Hörgerätewandler abliegenden Ankoppelende an der Ankoppelstelle anliegt, wird diese Aufgabe erfindungsgemäß dadurch gelöst, daß das Ankoppelende von einem Koppelelement mit einer Kontaktfläche gebildet ist, die eine an die Oberflächenform der Ankoppelstelle anpaßbare oder angepaßte Oberflächenform sowie eine solche Oberflächenbeschaffenheit und Oberflächengröße aufweist, daß es durch Anlegen des Ankoppelendes an die Ankoppelstelle zu einer dynamischen Zug-Druck-Kraftkopplung von Koppelelement und Ossikelkette durch Oberflächenadhäsion kommt, die für eine sichere gegenseitige Verbindung von Koppelelement und Ossikelkette ausreicht.

Die Vorrichtung nach der Erfindung sorgt für eine Ankopplung des ausgangsseitigen Wandlerteils eines in einer Mastoidhöhle implantierbaren elektromechanischen Hörgerätewandlers an ein Ossikel (Hammer, Amboß, Steigbügel; bevorzugt Amboß), an die Steigbügelfußplatte oder an eine das runde Fenster oder ein artifizielles Fenster abschließende Membran durch Oberflächenadhäsion. Unter dem Begriff "Oberflächenadhäsion" ist vorliegend eine Haftung unter dem Einfluß der bei hinreichend starker Annäherung an den Berührungsflächen wirksam werdenden molekularen Anziehungskräfte oder eine gegenseitige mechanische Verblockung der Berührungsflächen ohne den Einsatz eines Klebstoffes oder Zements zu verstehen. Zu einer entsprechenden Flächenhaftung können auch Luftblasen beitragen, die in Oberflächenvertiefungen eingeschlossen sind. Werden die Berührungsflächen Kräften ausgesetzt, welche die Flächen zu trennen suchen, erzeugen solche Luftblasen eine Saugwirkung, was die Trennung erheblich erschwert (Bild der Wissenschaft, April 1999, S. 10).

Ein grundlegender Vorteil einer solchen Adhäsionskopplung besteht darin, daß die Ankoppelstelle zum Beispiel des Ossikels nicht hauptsächlich in Schwingungsrichtung des antreibenden Wandlers "zwangsgeführt" wird, wobei eine solche "Zwangsführung" zu einer nichtoptimalen Schwingungsform der Steigbügelfußplatte im ovalen Fenster führen kann. (Eine vorzugsweise Schwingungsform ist eine kolbenförmige Schwingung der Steigbügelfußplatte senkrecht zu ihrer Ebene.) Vielmehr stellt das Ossikel bei Nutzung der Oberflächenadhäsionskopplung seine (frequenzabhängige) Schwingungsrichtung aufgrund der dynamischen Eigenschaften des intakten Mittelohres selbst ein. Dieser Vorteil gilt auch bei nicht-intakter, (teil-)abgebauter Ossikelkette und Ankopplung an den dem Innenohr zugewandten "Rest" der Kette, und im Extremfall auch bei nur verbleibendem Steigbügel oder nur Steigbügelfußplatte, da diese(r) durch das sogenannte Ligament (ein elastisches Ringband, das den Steigbügel im ovalen Fenster "hält") aufgehängt ist. Darüberhinaus ist eine postoperative Lösbarkeit der Ossikelankopplung auch noch nach mehrjähriger Körperverweilzeit gegeben. Eine solche Lösung der Verbindung ist mit adäquatem Kraftaufwand und unter Verwendung von ohrchirurgischen Standardinstrumenten möglich.

Es ist zwar bei einer externen (nicht implantierbaren) Hörhilfe mit elektromagetischem Ausgangswandler bekannt (EP-B-0 556 300), auf nicht invasivem Wege einen Permanentmagneten des Ausgangswandlers mittels manuell lösbarer Oberflächenhaftung an der Außenfläche des Trommelfells zu halten. In diesem Fall spielen aber Probleme, wie eine nicht optimale Schwingungsform der Steigbügelfußplatte durch Zwangsführung eines Ossikels und risikobehaftetes Arbeiten im Innenohr während einer Implantation, keine Rolle. Außerdem ist die an der Außenfläche des Trommelfells zur Verfügung stehende große Fläche nicht vergleichbar mit den winzigen Ankoppelflächen im Mittelohr.

Daneben ist eine passive Ossikelprothese mit einem Kopf, einem Schaft und einem biegbaren Zwischenstück bekannt (WO 90/11737). Das Zwischenstück erlaubt es, die Winkelausrichtung des Schafts mit Bezug auf den Kopf einzustellen. Bei implantierter Prothese stützt sich der Schaft an dem Steigbügelbogen, einem Steigbügelschenkel oder der Steigbügelfußplatte ab, während der Prothesenkopf am Trommelfell oder am Hammer unter einer solchen Vorspannung anliegt, daß das Trommelfell leicht gespannt wird. Die infolgedessen von dem Trommelfell auf die Prothese ausgeübte Druckkraft hält die Prothese an Ort und Stelle. Der Kopf und der Schaft der Prothese sind vorzugsweise aus Hydroxylapatit oder aus einem Gemisch von Hydroxylapatitteilchen und Silkon oder Polyurethan gefertigt. An einem solchen Werkstoff haftet menschliches Gewebe an, wodurch die Festlegung der Prothese im Mittelohr unterstützt werden soll.

Bevorzugte weitere Ausgestaltungen der Erfindung ergeben sich aus den Unteransprüchen.

Insbesondere weist die Koppelanordnung zweckmäßig eine mit dem ausgangsseitigen Wandlerteil fest verbundene, im implantierten Zustand von der Mastoidhöhle in die Paukenhöhle reichende Koppelstange und ein Koppelelement auf, das mit dem von dem ausgangsseitigen Wandlerteil abliegenden Ende der Koppelstange verbindbar oder verbunden ist und das Ankoppelende der Koppelanordnung bildet. Eine solche Koppelstange stellt ein mechanisch steifes Koppelorgan von verhältnismäßig geringer Masse dar, das an der Außenseite einer schwingfähigen Membran des elektromechanischen Hörgerätewandlers fest angebracht sein kann und das sich bei der Implantation durch den natürlichen oder artifiziell geringfügig erweiterten aditus ad antrum, durch eine Eröffnung des chorda-facialis-Winkels oder durch eine artifizielle Bohrung vom Mastoid aus in das Mittelohr schieben läßt, um dort über das Koppelelement mit der gewünschten Ankoppelstelle verbunden zu werden. Es wird dadurch sichergestellt, daß der vibratorische Stimulus des Wandlers weitgehend verlustfrei, das heißt schallhart, in das Zielossikel eingeleitet wird.

Die Koppelstange und das Koppelelement können über ein flexibles Zwischenglied miteinander verbunden sein, bei dem es sich um ein gesondertes Bauteil handeln kann oder das von dem Koppelelement selbst gebildet sein kann. Im letztgenannten Fall kann das Koppelelement zwecks Bildung des flexiblen Zwischengliedes einfach mit einer Einschnürung versehen sein. Das flexible Zwischenglied kann sich hinsichtlich seines Raumwinkels selbsttätig optimal einstellen oder durch den Operateur intraoperativ individuell optimal plastisch eingestellt werden.

Das flexible Zwischenglied kann vorteilhaft als Federelement ausgebildet sein und aus einer Metallegierrung mit Memory-Effekt oder sogenannter "Superelastizität", insbesondere Nitinol, bestehen. Der Einsatz eines derartigen Werkstoffes hat den Vorteil, daß die übertragene Kraft auch bei unterschiedlichen Stellwegen etwa gleich bleibt.

Die Koppelstange und das Koppelelement können aber auch über ein Kugelgelenk miteinander verbunden sein, um die oben geschilderte optimale Raumwinkeleinstellung zu erreichen.

Vorzugsweise ist die Koppelanordnung so gestaltet und/oder im implantierten Zustand so positioniert, daß das Ankoppelende die Ankoppelstelle ohne statische Vorspannung oder nur mit leichter Vorspannung berührt.

Zur sicheren Haftung durch Oberflächenadhäsion trägt bei, wenn mindestens im implantierten Zustand zwischen dem Ankoppelende und der Ankoppelstelle ein Feuchtigkeitsfilm ausgebildet ist. Dafür läßt sich ein natürlicher Feuchtigkeitsfilm nutzen, der auf die im Mittelohrraum vorliegende Feuchte von 100% zurückzuführen ist.

Das Ankoppelende kann vorteilhaft vor dem Ankoppeln mit Bezug auf die Ankoppelstelle konkav ausgebildet sein. Dadurch entsteht beim Anlegen des Ankoppelendes an die Ankoppelstelle ein Hohlraum, der durch leichtes Andrücken des Ankoppelendes zusammengedrückt wird. Der dabei ausgebildete Unterdruck unterstützt die Haftung.

Die Ankoppelstelle kann auch von einem gegebenenfalls anatomisch anpaßbaren Koppelplättchen gebildet sein, das im implantierten Zustand mit der Oberfläche des zu kontaktierenden Teils der Ossikelkette, der Steigbügelfußplatte oder einer das runde Fenster oder ein artifizielles Fenster abschließenden Membran fest verbunden, beispielsweise aufgeklebt oder aufzementiert, ist. Auf diese Weise läßt sich für eine noch besser definierte Adhäsionswirkung sorgen, weil eine dynamische Kraftübertragung zwischen zwei definierten Materialien und Geometrien stattfindet, die für die Adhäsionskopplung optimiert werden können. Die Kopplung ist damit auch in ihrer Wirkung reproduzierbarer als bei einer Direkteinwirkung auf ein Ossikel.

Verbesserte anatomische Einstellmöglichkeiten können unter Umständen dadurch erreicht werden, daß das Koppelelement am Ankoppelende eine großflächige, durchbrochene Struktur hat und/oder mit mehreren federnden Armen versehen ist.

Bevorzugte Ausführungsbeispiele der Anordnung nach der Erfindung werden nachstehend anhand der beiliegenden Zeichnungen näher erläutert. Es zeigen:
- Fig. 1: eine Schnittansicht eines Teils des menschlichen Schädels mit implantiertem elektromechanischem Hörgerätewandler und einer bevorzugten Ausführungsform der Vorrichtung zum mechanischen Ankoppeln des ausgangsseitigen Wandlerteils an die Ossikelkette,
- Fig. 2: in größerem Maßstab eine perspektivische Darstellung des Koppelelements und eines Teils der Koppelstange der Koppelanordnung nach Fig. 1,
- Fig. 3: einen Schnitt der Koppelanordnung nach Fig. 2,
- Fign. 4 und 5: eine perspektivische Darstellung und einen Schnitt entsprechend den Figuren 2 und 3 für eine abgewandelte Ausführungsform der Koppelanordnung,
- Fign. 6 und 7: eine perspektivische Darstellung und einen Schnitt entsprechend den Figuren 2 und 3 für eine weiter abgewandelte Ausführungform der Koppelanordnung,
- Fign. 8 und 9: eine perspektivische Darstellung und einen Schnitt entsprechend den Figuren 2 und 3 für eine weiter abgewandelte Ausführungsform der Koppelanordnung,
- Fig. 10: eine Teilseitenansicht der am Amboßkörper anliegenden Koppelanordnung gemäß den Figuren8 und 9,
- Fig. 11: einen Schnitt einer Koppelanordnung mit zweiteiligem Koppelelement,
- Fign. 12 und 13: eine perspektivische Darstellung und einen Schnitt entsprechend den Figuren 2 und 3 für eine weiter abgewandelte Ausführungform der Koppelanordnung,
- Fig. 14: eine Teilschnittansicht der am Amboßkörper anliegenden Koppelanordnung gemäß den Figuren 12 und 13,
- Fign. 15 und 16: eine perspektivische Darstellung und einen Schnitt entsprechend den Figuren 2 und 3 für eine weiter abgewandelte Ausführungsform der Koppelanordnung,
- Fig. 17: eine perspektivische Darstellung der in den Figuren 15 und 16 gezeigten Koppelanordnung mit im Zuge der Implantation zurechtgebogenem Zwischenglied,
- Fig. 18: eine Teilschnittansicht der am Amboßkörper anliegenden Koppelanordnung gemäß den Figuren 15 bis 17,
- Fign. 19 und 20: einen Schnitt und eine perspektivische Darstellung entsprechend den Figuren 3 und 2 für eine weiter abgewandelte Ausführungsform der Koppelanordnung,
- Fig. 21: eine Teilschnittansicht der am Amboßkörper anliegenden Koppelanordnung gemäß den Figuren 19 und 20,
- Fig. 22: ein Spannungs-Dehnungsdiagramm des bei der Koppelanordnung gemäß den Figuren 19 bis 21 bevorzugt vorzusehenden Federmaterials (Nitinol),
- Fign. 23 und 24: einen Schnitt und eine perspektivische Darstellung entsprechend den Figuren 3 und 2 für eine weiter abgewandelte Ausführungsform der Koppelanordnung,

- Fign. 25 und 26: einen Schnitt und eine perspektivische Darstellung entsprechend den Figuren 3 und 2 für eine weiter abgewandelte Ausführungsform der Koppelanordnung,
- Fign. 27 und 28: einen Schnitt und eine perspektivische Darstellung entsprechend den Figuren 3 und 2 für eine weiter abgewandelte Ausführungform der Koppelanordnung,
- Fign. 29 und 30: einen Schnitt und eine perspektivische Darstellung entsprechend den Figuren 3 und 2 für eine weiter abgewandelte Ausführungsform der Koppelanordnung,
- Fign. 31 und 32: einen Schnitt und eine perspektivische Darstellung entsprechend den Figuren 3 und 2 für eine weiter abgewandelte Ausführungsform der Koppelanordnung,
- Fign. 33 und 34: einen Schnitt und eine perspektivische Darstellung entsprechend den Figuren 3 und 2 für eine weiter abgewandelte Ausführungsform der Koppelanordnung sowie
- Fig. 35: eine Seitenansicht der am Amboßkörper anliegenden Koppelanordnung gemäß den Figuren33 und 34.

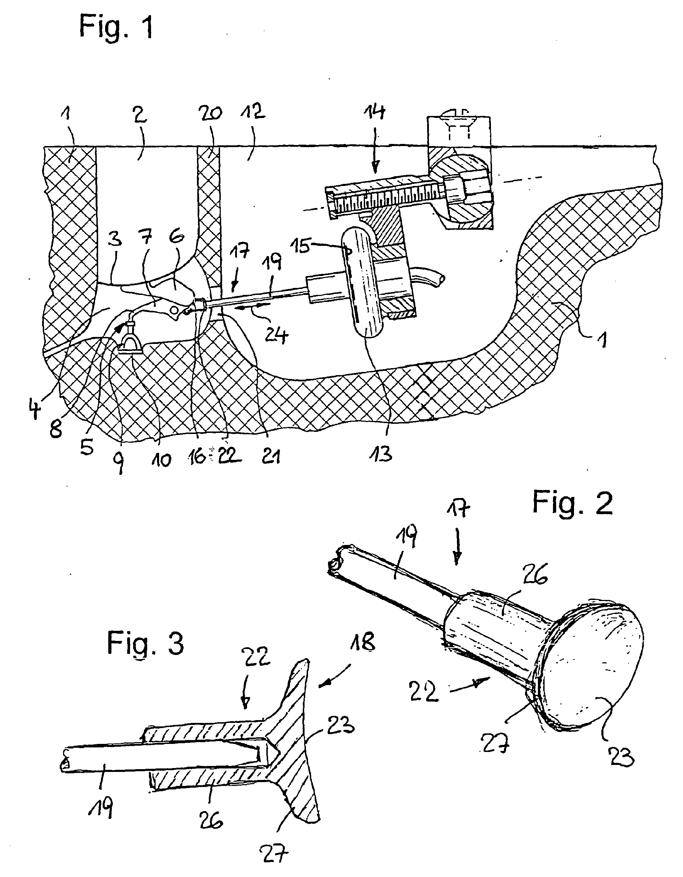

In Fig. 1 ist ein Teil eines menschlichen Schädelknochens 1 mit dem Gehörgang 2, dem davon durch das Trommelfell 3 abgetrennten Mittelohrraum (Paukenhöhle) 4 und der in der Paukenhöhle befindlichen Ossikelkette 5 dargestellt, zu welcher der Hammer 6, der Amboß 7 mit dem Amboßfortsatz 8 sowie der Steigbügel 9 mit der Steigbügelfußplatte 10 gehören. In einer artifiziellen Mastoidhöhle 12 ist ein elektromechanischer Hörgerätewandler 13 mittels eines insgesamt mit 14 bezeichneten Positionier- und Fixiersystems fixiert. Der Hörgerätewandler 13 kann beispielsweise als Piezowandler zur vibratorischen Stimulation der Ossikelkette insbesondere in der aus US-A-5 277 694 bekannten Weise aufgebaut sein, und er ist Bestandteil eines mindestens teilimplantierbaren und vorzugsweise vollimplantierbaren Hörgerätes, beispielsweise eines Hörgerätes der aus HNO 1997 Vol. 45, 749 -774 bekannten Art.

Zum mechanischen Ankoppeln eines zu mechanischen Schwingungen anregbaren, in Fig. 1 nur schematisch angedeuteten ausgangsseitigen Wandlerteils 15 des Hörgerätewandlers 13 an eine vorgewählte Ankoppelstelle 16 an der Ossikelkette 5, beispielsweise an den "glatten" Körper des Ambosses 7, von Mastoidseite aus, ist eine biokompatible, mechanisch passive Koppelanordnung 17 vorgesehen, die mit dem aktiv schwingfähigen ausgangsseitigen Wandlerteil 15 verbunden ist und die im implantierten Zustand mit einem von dem Hörgerätewandler 13 abliegenden Ankoppelende 18 an der Ankoppelstelle 16 anliegt. Wird an den Hörgerätewandler 13 eine elektrische Spannung angelegt, wird die Koppelanordnung 17 mittels des ausgangsseitigen Wandlerteils 15 zu vibratorischen Schwingungen in Axialrichtung der Koppelanordnung veranlaßt. Infolgedessen führen die von einem (nicht dargestellten) eingangsseitigen Wandler (Mikrofon) aufgenommenen und elektrisch gewandelten Audiosignale nach elektronischer Verstärkung in einem Elektronikmodul des Hörgeräts unmittelbar zu mechanischen Auslenkungen der Koppelanordnung 17. Diese Auslenkungen entsprechen der akustischen Information. Die Auslenkungen der Koppelanordnung 17 werden an die Ossikelkette des Mittelohrs bzw. an den Steigbügel oder das ovale bzw. runde Fenster weitergeleitet. Sie bewirken so bei entsprechender Auslegung des vorverarbeitenden elektronischen Systems einen audiologischen Verstärkungseffekt.

Die Koppelanordnung 17 weist im veranschaulichten Ausführungsbeispiel eine mit dem ausgangsseitigen Wandlerteil 15 mechanisch fest verbundene Koppelstange 19 auf, die im veranschaulichten Ausführungsbeispiel im wesentlichen auf ihrer gesamten Länge die Gestalt eines geraden Zylinders hat und die im implantierten Zustand von der Mastoidhöhle 12 aus durch einen in der hinteren Gehörgangswand 20 befindlichen natürlichen Knochendurchbruch (Aditus ad antrum) 21 hindurch in die Paukenhöhle 4 reicht. Zu der Koppelanordnung 17 gehört ferner ein Koppelelement 22, das mit dem von dem Hörgerätewandler 13 abliegenden Ende der Koppelstange 19 verbunden ist und das Ankoppelende 18 der Koppelanordnung 17 bildet. Das Ankoppelende 18 hat eine Kontaktfläche 23, die eine an die Oberflächenform der Ankoppelstelle 16 angepaßte Oberflächenform sowie eine solche Oberflächenbeschaffenheit und Oberflächengröße aufweist, daß es durch Anlegen des Ankoppelendes 18 an die Ankoppelstelle 16 zu einer dynamischen Zug-Druck-Kraftkopplung von Koppelelement 22 und Zielossikel (in dem veranschaulichten Fall dem Amboß 7) durch eine Oberflächenadhäsion kommt, die für eine spielfreie gegenseitige Verbindung von Koppelelement 22 und Ossikelkette 5 ausreicht. Die Adhäsionswirkung wird dabei durch den Umstand unterstützt, daß der Mittelohrraum 4 immer 100%-Feuchte aufweist und infolgedessen auf den Ossikeln 6, 7, 9 ein natürlicher Feuchtigkeitsfilm vorhandenen ist. Das Koppelelement 22 ist in seiner Grundform entsprechend dem Zielossikel bzw. dem Ortsteil des Zielossikels an der Ankoppelstelle 16 so gestaltet (konkav, konvex oder plan), daß es das Ossikel ohne statische Vorspannung oder mit leichter Vorspannung berührt und eine dynamische Zug-Druck-Kraftkopplung aufgrund der entstehenden Adhäsion bewirkt. Eine gewollte statische Vorspannung gegenüber den Aufhängungsbändern des Mittelohres kann durch einen zweckentsprechenden Vorschub des Wandlers 13 und damit der Koppelstange 19 (entsprechend dem Doppelpfeil 24 in Fig. 1) mittels des Positionier- und Fixiersystems 14 erzeugt werden. Dafür geeignete Positionier- und Fixiersysteme sind in US-A-5 788 711 und in der älteren EP-Patentanmeldung 99 122 684.6 beschrieben.

Das in größerem Maßstab in den Figuren 2 und 3 gezeigte Koppelelement 22 weist einen hülsenförmigen Abschnitt 26 und einen damit einstückig verbundenen, das Ankoppelende 18 der Koppelanordnung 17 darstellenden Flanschabschnitt 27 auf, der mit einer konkaven Kontaktfläche 23 versehen ist. Das Koppelelement 22 ist mit seinem hülsenförmigen Abschnitt 26 auf das von dem Wandler 13 abliegende Ende der Koppelstange 19 aufgesteckt und mit der Koppelstange fest verbunden, beispielsweise vercrimpt, verschweißt, verlötet oder auch verklebt.

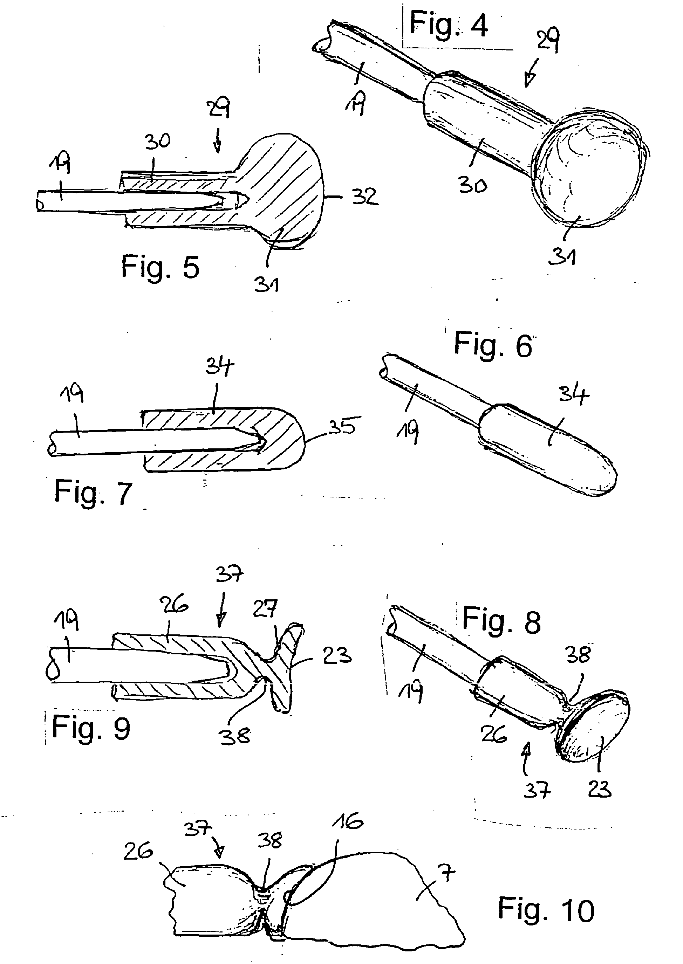

In den Figuren 4 und 5 ist ein Koppelelement 29 dargestellt, das sich von dem Koppelelement 22 nur dadurch unterscheidet, daß an einen hülsenförmigen Abschnitt 30 ein das Ankoppelende bildender, kugeliger Kopf 31 anschließt. Der Kopf 31 hat einen größeren Durchmesser als der Abschnitt 30, und er weist konvex gekrümmte Kontaktfläche 32 auf.

Die Figuren 6 und 7 zeigen ein auf das frei Ende der Koppelstange 19 aufgestecktes Koppelelement 34 mit im wesentlichen konstantem Außendurchmesser und halbkugeligem Ankoppelende 35.

Das in den Figuren 8, 9 und 10 veranschaulichte Koppelelement 37 ist ähnlich dem Koppelelement 22 der Figuren 2 und 3. Es weist jedoch zusätzlich ein flexibles Zwischenglied in Form einer Einschnürung 38 an der Übergangsstelle zwischen dem hülsenförmigen Abschnitt 26 und dem Flanschabschnitt 27 auf. Je nach dem im Einzelfall für das Koppelelment gewählten Werkstoff und der Dimensionierung der Einschnürung 38 kann sich dadurch die Kontaktfläche 23 selbsttätig im Raumwinkel optimal zur Ankoppelstelle 16 einstellen, oder das Koppelelment kann durch den Operateur intraoperativ individuell optimal plastisch verformt werden.

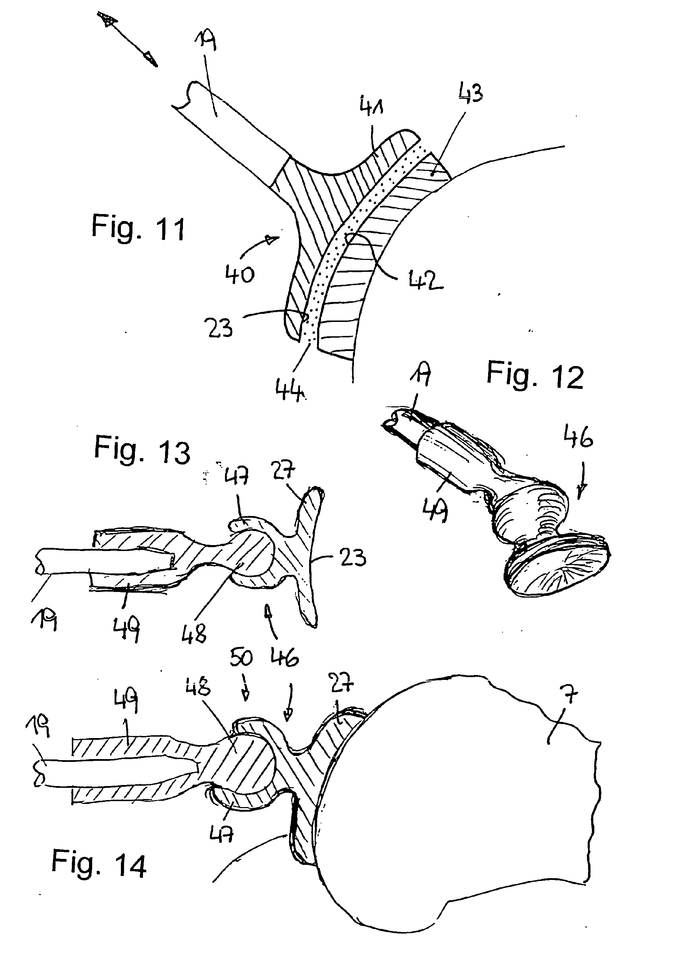

Bei der Ausführungsform der Fig. 11 weist ein Koppelelment 40 ähnlich wie im Falle des Koppelelments der Figuren 2 und 3 einen relativ großflächigen Flanschabschnitt 41 mit konkaver Kontaktfläche 23 auf. Der konkaven Kontaktfläche 23 liegt eine komplementär konvex gekrümmte Fläche 42 eines dünnen Koppelplättchens 43 gegenüber, das im gezeigten Beispiel mit der Oberfläche des Anboßkörpers fest verbunden ist. Das Kuppelplättchen 43 kann bedarfsweise anatomisch angepaßt werden. Zwischen den Flächen 23 und 42 befindet sich vorzugsweise ein Flüssigkeitsfilm 44. Eine solche Anordnung erlaubt es, eine noch besser definierte und noch besser reproduzierbare Adhäsionswirkung als bei Direkteinwirkung auf ein Ossikel zu erzielen, weil die dynamische Kraftübertragung zwischen zwei definierten Materialien und Geometrien stattfindet. Bei der gezeigten Ausführungsform ist das Koppelelment 40 mit der Koppelstange 19 stumpf verbunden. Stattdessen kann das freie Koppelstangenende auch von einem an den Flanschabschnitt 41 anschließenden hülsenförmigen Abschnitt entsprechend den Figuren 5, 7 und 9 aufgenommen werden.

Die in Verbindung mit der Ausführungsform der Figuren 8 bis 10 erläuterte Einstellung der Kontaktfläche kann auch durch ein Pivot-Element gemäß den Figuren 12 bis 14 erreicht werden. In diesem Fall ist ein Koppelelement 46 vorgesehen, bei dem sich an den die Kontaktfläche 23 bildenden Flanschabschnitt 27 eine Kugelaufnahme 47 anschließt. In die Kugelaufnahme 47 greift ein Kugelkopf 48 ein, der Teil eines mit der Koppelstange 19 fest verbundenen Kugelgelenkteils 49 ist. Das aus der Kugelaufnahme 47 und dem Kugelgelenkteil 49 bestehende Kugelgelenk 50 erlaubt nicht nur eine Einstellung des Raumwinkels der Kontaktfläche 23 mit Bezug auf die Längsachse der Koppelstange 19, sondern zusätzlich auch eine Drehung der Koppelstange 19 gegenüber dem Koppelelement 46. Es versteht sich, daß abweichend von der gezeigten Ausführungsform auch das mit der Koppelstange 19 fest verbundene Kugelgelenkteil als Kugelaufnahme ausgebildet sein kann, die mit einem Kugelkopf zusammenwirkt, der Teil des Koppelelements ist. Des weiteren kann gegebenenfalls auf ein eigenes Zwischenteil zwischen der Koppelstange und dem Koppelelement verzichtet werden, indem der Kugelkopf beziehungsweise die Kugelaufnahme unmittelbar an der Koppelstange angeformt wird.

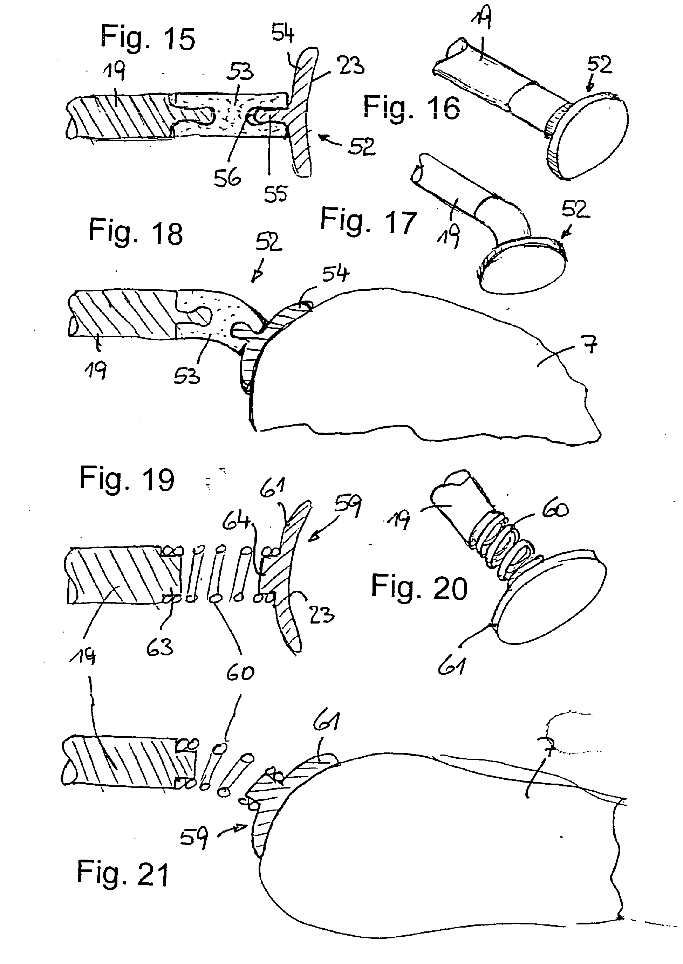

Die Figuren 15 bis 18 zeigen eine Ausführungsform, bei welcher zwischen die Koppelstange 19 und ein Koppelelement 52 ein flexibles Zwischenglied 53 in Form eines gesonderten Bauteils eingefügt ist, um den Raumwinkel der Kontaktfläche 23 einstellen zu können. Das Koppelelement 52 weist einen relativ großflächigen Flanschabschnitt 54 mit einem Stutzen 55 auf, der von der der Kontaktfläche 23 gegenüberliegenden Oberfläche des Koppelelements 52 absteht. Das freie Ende des Stutzens 55 ist leicht ballig und rastet in eine komplementäre Ausnehmung 56 am einen Ende des Zwischengliedes 53 ein. In entsprechender Weise ist das andere Ende des Zwischengliedes 53 mit der Koppelstange 19 verbunden.

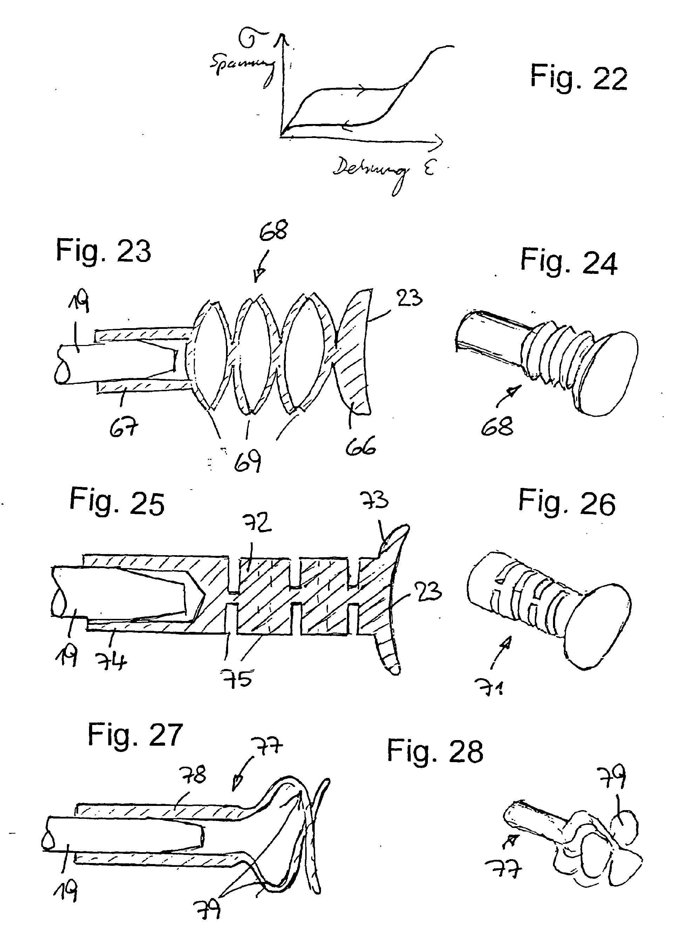

Im Falle der Ausführungsform des Koppelelements 59 gemäß den Figuren 19 bis 21 ist als flexibles Zwischenglied eine Schraubenfeder 60 vorgesehen, die einen Flanschabschnitt 61 des Koppelelements mit der Koppelstange 19 verbindet. Die Enden der Schraubenfeder 60 sind mindestens kraftschlüssig mit Federabstützungen 63 und 64 der Koppelstange 19 beziehungsweise des Flanschabschnitts 61 verbunden. Die Feder 60 kann gegebenenfalls aus einer Metallegierung mit Memory-Effekt, insbesondere Nitinol, gefertigt sein. Eine solche Metallegierung kann sich auch durch sogenannte "Superelastizität" auszeichnen, das heißt, die übertragene Kraft bleibt, wie aus dem Spannungs/Dehnungs-Diagramm der Fig. 22 hervorgeht, in einem gewissen Bereich auch bei unterschiedlichen Stellwegen etwa gleich.

Weitere mögliche Federelemente sind in den Figuren 23 bis 28 dargestellt.

Bei der Ausführungsform gemäß den Figuren 23 und 24 befinden sich zwischen einem die Kontaktfläche 23 bildenden Flanschabschnitt 66 und einem hülsenförmigen Abschnitt 67 eines Koppelelements 68 drei Tellerfederabschnitte 69.

Im Falle der in den Figuren 25 und 26 dargestellten Ausführungsform ist ein Koppelelement 71 mit einem zylindrischer Abschnitt 72 versehen, der zwischen einem die Kontaktfläche 23 bildenden Flanschabschnitt 73 und einem hülsenförmigen Abschnitt 74 eingefügt ist. In dem zylindrischen Abschnitt 72 ist eine Folge von in Umfangsrichtung um 90° versetzten Einkerbungen 75 vorgesehen, wodurch dem zylindrischen Abschnitt 72 Federeigenschaften verliehen werden.

Bei dem Koppelelement 77 nach den Figuren 27 und 28 geht ein hülsenförmiger Abschnitt 78 in eine Anordnung von drei in Umfangsrichtung gegeneinander versetzten Federbügeln 79 über.

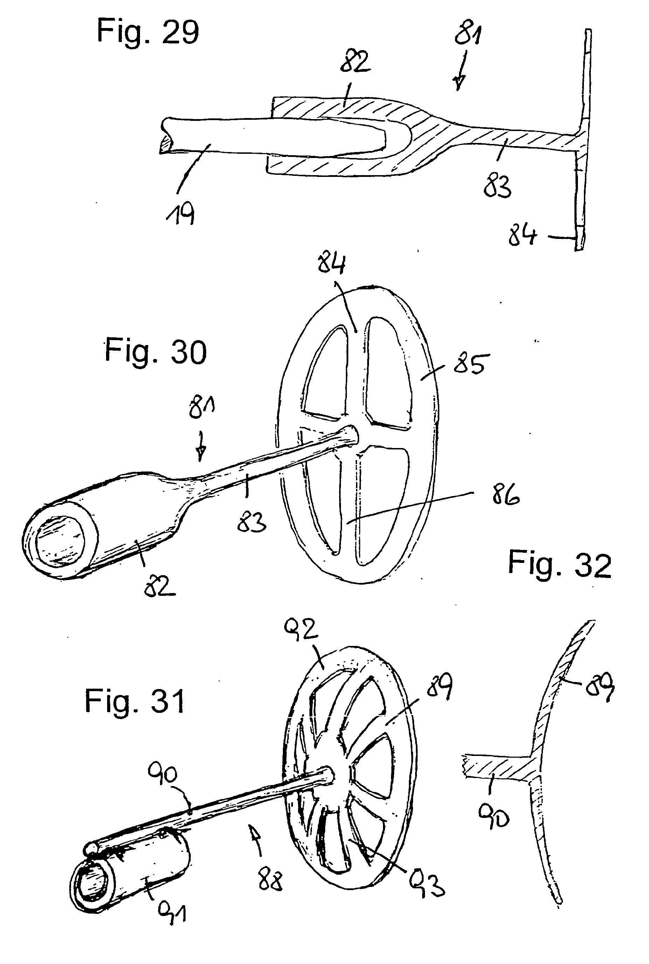

Das in den Figuren 29 und 30 veranschaulichte Koppelelement 81 weist einen hülsenförmigen Abschnitt 82 auf, der über einen zum Abschnitt 82 konzentrischen, schlanken Schaft 83 mit einem planen Adhäsionselement 84 verbunden ist. Das Adhäsionselement 84 hat eine durchbrochene Struktur mit einem Außenring 85 und mehreren Speichen 86.

Das Koppelelement 88 des Ausführungsbeispiels der Figuren 31 und 32 ist mit einem durchbrochenen konkaven Adhäsionselement 89 versehen. Das Adhäsionselement 89 ist über einen Schaft 90 mit einem auf die Koppelstange aufschiebbaren hülsenförmigen Abschnitt 91 verbunden, der seitlich an dem Schaft 90 befestigt ist. Zu dem Adhäsionselement 89 gehören wiederum ein Außenring 92 und eine Mehrzahl von Speichen 93. Die Außenringe 85, 92 können für eine bessere anatomische Einstellmöglichkeit in nicht näher dargestellter Weise ebenfalls durchbrochen (geschlitzt) sein.

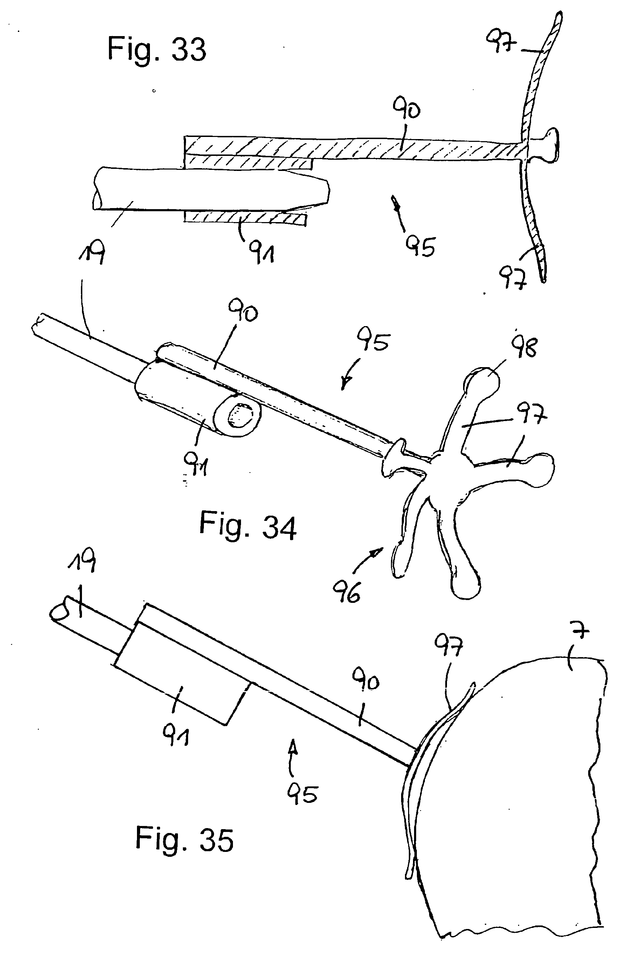

Durch gänzliches Weglassen des Außenrings 85 beziehungsweise 92 kommt man zu einem Koppelelement 95 der in den Figuren 33 bis 35 gezeigten Art, bei dem ein Adhäsionselement 96 eine Mehrzahl von abstehenden Armen 97 aufweist. Die freien Enden 98 der Arme 97 bilden gemeinsam die Kontaktfläche.

Die beschriebene Koppelanordnung kann grundsätzlich aus beliebigen biokompatiblen Werkstoffen, insbesondere Metallen, Metallegierungen und/oder Kunststoffen, bestehen. Als metallische Werkstoffe kommen vor allem Titan, Gold, Silber, Niob, Tantal, Platin, Platin-Iridium, oder Legierungen dieser Metalle, Implantatstahl, NiTi (Nitinol) oder andere biokompatible Gedächtnisformmetalle in Betracht. Bei den Kunststoffen kann es sich vor allem um Silikone, Polyurethane, PTFE, FEP, Polycarbonate und dergleichen handeln. Diese breite Auswahl insbesondere an Kunststoffen ist entsprechend einzuengen, wenn spezielle Materialeigenschaften, wie plastische Verformbarkeit zur individuellen Anpassung an das Zielossikel, erforderlich sind.

Bei der Materialauswahl und der konstruktiven Gestaltung ist darauf zu achten, daß es darauf ankommt, den vibratorischen Stimulus (Aktio) des Wandlers 13 möglichst verlustfrei, das heißt schallhart, in das Zielossikel einzuleiten. Dabei soll die Gesamtmasse der Koppelanordnung vorzugsweise unter der Masse des Amboß liegen, die durchschnittlich 25 mg beträgt. Ein geringstmögliches Gewicht der Koppelanordnung führt zudem zur Reduzierung von Trägheitskräften bei Beschleunigung durch externe Einflüsse wie Schlag, Vibration und dergleichen.

## Patentansprüche

1. Vorrichtung zum mechanischen Ankoppeln eines zu mechanischen Schwingungen anregbaren ausgangsseitigen Wandlerteils (15) eines außerhalb des Mittelohrbereiches in einer artifiziellen Mastoidhöhle implantierbaren elektromechanischen Hörgerätewandlers (13) an eine vorgewählte Ankoppelstelle (16) an der Ossikelkette, der Steigbügelfußplatte oder einer das runde Fenster oder ein artifizielles Fenster in der Cochlea, im Vestibulum oder im Labyrinth (Gleichgewichtsorgan) abschließenden Membran, mit einer biokompatiblen, mechanisch passiven Koppelanordnung (17), die mit dem ausgangsseitigen Wandlerteil verbunden ist und die im implantierten Zustand von der Mastoidhöhle in die Paukenhöhle reicht sowie mit einem von dem Hörgerätewandler abliegenden Ankoppelende (18) an der Ankoppelstelle anliegt, **dadurch gekennzeichnet, daß** das Ankoppelende (18) von einem Koppelelement (22, 29, 34, 37, 40, 46, 52, 59, 68, 71, 77, 81, 88, 95) mit einer Kontaktfläche (32) gebildet ist, die eine an die Oberflächenform der Ankoppelstelle anpaßbare oder angepaßte Oberflächenform sowie eine solche Oberflächenbeschaffenheit und Oberflächengröße aufweist, daß es durch Anlegen des Ankoppelendes an die Ankoppelstelle zu einer dynamischen Zug-Druck-Kraftkopplung von Koppelelement und Ossikelkette durch Oberflächenadhäsion kommt, die für eine sichere gegenseitige Verbindung von Koppelelement und Ossikelkette ausreicht.

2. Vorrichtung nach Anspruch 1, **dadurch gekennzeichnet, daß** die Koppelanordnung (17) eine mit dem ausgangsseitigen Wandlerteil (15) fest verbundene, im implantierten Zustand von der Mastoidhöhle in die Paukenhöhle reichende Koppelstange (19) und ein Koppelelement (22, 29, 34, 37, 40, 46, 52, 59, 68, 71, 77, 81, 88, 95) aufweist, das mit dem von dem ausgangsseitigen Wandlerteil abliegenden Ende der Koppelstange verbindbar oder verbunden ist und das Ankoppelende (18) der Koppelanordnung bildet.

3. Vorrichtung nach Anspruch 2, **dadurch gekennzeichnet, daß** die Koppelstange (19) und das Ankoppelende (18) über ein flexibles Zwischenglied (38, 53, 60, 69, 72, 79) miteinander verbunden sind.

4. Vorrichtung nach Anspruch 3, **dadurch gekennzeichnet, daß** das flexible Zwischenglied (38, 69, 72, 79) von dem Koppelelement (37, 68, 71, 77) selbst gebildet ist.

5. Vorrichtung nach Anspruch 4, **dadurch gekennzeichnet, daß** das Koppelelement (37) zwecks Bildung des flexiblen Zwischengliedes mit einer Einschnürung (38) versehen ist.

6. Vorrichtung nach Anspruch 3, **dadurch gekennzeichnet, daß** das flexible Zwischenglied (53, 60) ein gesondertes Bauteil ist.

7. Vorrichtung nach einem der Ansprüche 3 bis 6, **dadurch gekennzeichnet, daß** das flexible Zwischenglied als Federelement (60, 69, 79) ausgebildet ist.

8. Vorrichtung nach Anspruch 7, **dadurch gekennzeichnet, daß** das flexible Zwischenglied (38, 53, 60, 69, 72, 79) aus einer Metallegierrung mit Memory-Effekt besteht.

9. Vorrichtung nach Anspruch 8, **dadurch gekennzeichnet, daß** das flexible Zwischenglied (38, 53, 60, 69, 72, 79) aus Nitinol gefertigt ist.

10. Vorrichtung nach einem der Ansprüche 2 bis 5, **dadurch gekennzeichnet, daß** die Koppelstange (19) und das Koppelelement (46) über ein Kugelgelenk (50) miteinander verbunden sind.

11. Vorrichtung nach einem der vorhergehenden Ansprüche, **dadurch gekennzeichnet, daß** die Koppelanordnung (17) so gestaltet ist und/oder im implantierten Zustand so positioniert ist, daß das Ankoppelende (18) die Ankoppelstelle (16) ohne statische Vorspannung berührt.

12. Vorrichtung nach einem der Ansprüche 1 bis 10, **dadurch gekennzeichnet, daß** die Koppelanordnung (17) so gestaltet ist und/oder im implantierten Zustand so positioniert ist, daß das Ankoppelende (18) die Ankoppelstelle (16) mit leichter Vorspannung berührt.

13. Vorrichtung nach einem der vorhergehenden Ansprüche, **dadurch gekennzeichnet, daß** mindestens im implantierten Zustand zwischen dem Ankoppelende (18) und der Ankoppelstelle (16) ein Feuchtigkeitsfilm (44) ausgebildet ist.

14. Vorrichtung nach einem der vorhergehenden Ansprüche, **dadurch gekennzeichnet, daß** das Ankoppelende (18) vor dem Ankoppeln mit Bezug auf die Ankoppelstelle (16) konkav ausgebildet ist.

15. Vorrichtung nach einem der vorhergehenden Ansprüche, **dadurch gekennzeichnet, daß** die Ankoppelstelle von einem Koppelplättchen (43) gebildet ist, das im implantierten Zustand mit der Oberfläche des zu kontaktierenden Teils der Ossikelkette, der Steigbügelfußplatte oder einer das runde Fenster oder ein artifizielles Fenster abschließenden Membran fest verbunden ist.

16. Vorrichtung nach einem der vorhergehenden Ansprüche, **dadurch gekennzeichnet, daß** das Koppelelement (81, 88) am Ankoppelende eine großflächige, durchbrochene Struktur hat.

17. Vorrichtung nach einem der Ansprüche 1 bis 15, **dadurch gekennzeichnet, daß** das Koppelelement (95) am Ankoppelende mit mehreren federnden Armen (97) versehen ist.

## Claims

1. Device for mechanical coupling of an output-side transducer part (15) of an electromechanical hearing aid transducer (13), which is implantable in an artificial mastoid cavity outside the region of the middle ear, the transducer part having a capacity to be excited to mechanical vibrations, to a preselected coupling site (16) on the ossicular chain, the footplate of the stapes, or a membrane which closes the round window or an artificial window in the cochlea, in the vestibulum or in the labyrinth (equilibrium organ), comprising a biocompatible, mechanically passive coupling arrangement (17) which is connected to the output-side transducer part and which, in the implanted state, reaches from the mastoid cavity into the tympanic cavity and adjoins the coupling site with a coupling end (18) that faces away from the hearing aid transducer, **characterized in that** the coupling end (18) is formed by a coupling element (22, 29, 34, 37, 40, 46, 52, 59, 68, 71, 77, 81, 88, 95) with a contact surface (32) which has a surface shape which is matched or which can be matched to the surface shape of the coupling site and has a surface texture and surface size such that by placing the coupling end against the coupling site dynamic tension-compression force coupling of the coupling element and the ossicular chain occurs by surface adhesion which is sufficient for reliable mutual connection of the coupling element and the ossicular chain.

2. Device as claimed in claim 1, **characterized in that** the coupling arrangement (17) comprises a coupling rod (19) which, in the implanted state, reaches from the mastoid cavity into the tympanic cavity and is securely joined to the output-side transducer part (15), and a coupling element (22, 29, 34, 37, 40, 46, 52, 59, 68, 71, 77, 81, 88, 95) which can be connected or is connected to the end of the coupling rod facing away from the output-side transducer part and forms the coupling end (18) of the coupling arrangement.

3. Device as claimed in claim 2 **characterized in that** the coupling rod (19) and the coupling end (18) are joined to one another via a flexible intermediate element (38, 53, 60, 69, 72, 79).

4. Device as claimed in claim 3, **characterized in that** the flexible intermediate element (38, 69, 72, 79) is formed by the coupling element (37, 68, 71, 77) itself.

5. Device as claimed in claim 4, **characterized in that** the coupling element (37) is provided with a constriction (38) so as to form the flexible intermediate element.

6. Device as claimed in claim 3, **characterized in that** the flexible intermediate element (53, 60) is a separate component.

7. Device as claimed in any one of claims 3 to 6, **characterized in that** the flexible intermediate element is designed as a spring element (60, 69, 79).

8. Device as claimed in claim 7, **characterized in that** the flexible intermediate element (38, 53, 60, 69, 72, 79) is made of a metal alloy with memory effect.

9. Device as claimed in claim 8, **characterized in that** the flexible intermediate element (38, 53, 60, 69, 72, 79) is made ofnitinol.

10. Device as claimed in any one of claims 2 to 5, **characterized in that** the coupling rod (19) and the coupling element (46) are joined to one another via a ball and socket joint (50).

11. Device as claimed in claim 1, **characterized in that** the coupling arrangement (17) is designed and/or in the implanted state is positioned such that the coupling end (18) touches the coupling site (16) without static bias.

12. Device as claimed in any one of claims 1 to 10, **characterized in that** the coupling arrangement (17) is designed and/or in the implanted state is positioned such that the coupling end (18) touches the coupling site (16) with slight static bias.

13. Device as claimed in any one of the preceding claims, **characterized in that**, at least in the implanted state, a film of moisture (44) is formed between the coupling end (18) and the coupling site (16).

14. Device as claimed in any one of the preceding claims, **characterized in that** the coupling end (18) is designed to be concave with respect to the coupling site (16) prior to coupling.

15. Device as claimed in any one of the preceding claims, **characterized in that** the coupling site is formed by a coupling plate (43) which, in the implanted state, is securely joined to the surface of the part of the ossicular chain, the footplate of the stapes, or a membrane which closes the round window or an artificial window, to which contact is to be made.

16. Device as claimed in any one of the preceding claims, **characterized in that** the coupling element (81, 88) has a large-area, open-worked structure at the coupling end.

17. Device as claimed in any one of claims 1 to 15, **characterized in that** the coupling element (95) is provided with a plurality of resilient arms (97) at the coupling end.

## Revendications

1. Dispositif pour le couplage mécanique d'une partie transducteur (15) côté sortie, pouvant être excitée pour obtenir des vibrations mécaniques, d'un transducteur électromécanique de prothèse auditive (13) implantable à l'extérieur de la zone de l'oreille moyenne dans une cavité de mastoïde artificielle en un point de couplage (16) présélectionné sur la chaîne d'osselets, la plaque de base à étrier ou une membrane terminant la fenêtre ronde ou une fenêtre artificielle dans la cochlée, dans le vestibule ou le labyrinthe (organe d'équilibre), avec un dispositif de couplage (17) biocompatible, mécaniquement passif, qui est relié à la partie transducteur côté sortie, va dans l'état implanté de la cavité de mastoïde à la cage du tympan et s'appuie par une extrémité de couplage (18), éloignée du transducteur de prothèse auditive, sur le point de couplage, **caractérisé en ce que** l'extrémité de couplage (18) est formé par un élément de couplage (22, 29, 34, 37, 40, 46, 52, 59, 68, 71, 77, 81, 88, 95) avec une surface de couplage (32), qui présente une forme de surface pouvant être adaptée ou adaptée à la forme de surface du point de couplage et une qualité de surface et une grandeur de surface telles que, par le placement de l'extrémité de couplage au point de couplage, on arrive à un couplage de force de traction et de pression dynamique de l'élément de couplage et de la chaîne d'osselets par adhésion de surface qui est suffisant pour une liaison sûre et réciproque de l'élément couplage et de la chaîne d'osselets.

2. Dispositif selon la revendication 1, **caractérisé en ce que** le dispositif de couplage (17) présente une tige de couplage (19) reliée de façon fixe à la partie transducteur (15) côté sortie, allant dans l'état implanté de la cavité de mastoïde à la cage de tympan et un élément de couplage (22, 29, 34, 37, 40, 46, 52, 59, 68, 71, 77, 81, 88, 95) qui peut être relié ou est relié à l'extrémité, opposée à la partie transducteur côté sortie, de la tige de couplage et forme l'extrémité de couplage (18) du dispositif de couplage.

3. Dispositif selon la revendication 2, **caractérisé en ce que** la tige de couplage (19) et l'extrémité de couplage (18) sont reliées entre elles au moyen d'un élément intermédiaire (38, 53, 60, 69, 72, 79) flexible.

4. Dispositif selon la revendication 3, **caractérisé en ce que** l'élément intermédiaire (38, 69, 72, 79) flexible est formé par l'élément de couplage (37, 68, 71, 77) même.

5. Dispositif selon la revendication 4, **caractérisé en ce que** l'élément de couplage (37) est doté d'un rétrécissement (38) pour former l'élément intermédiaire flexible.

6. Dispositif selon la revendication 3, **caractérisé en ce que** l'élément intermédiaire (53, 60) flexible est un composant séparé.

7. Dispositif selon l'une quelconque des revendications 3 à 6, **caractérisé en ce que** l'élément intermédiaire flexible est conçu comme un élément de ressort (60, 69, 79).

8. Dispositif selon la revendication 7, **caractérisé en ce que** l'élément intermédiaire (38, 53, 60, 69, 72, 79) flexible est à base d'un alliage métallique avec effet de mémoire.

9. Dispositif selon la revendication 8, **caractérisé en ce que** l'élément intermédiaire (38, 53, 60, 69, 72, 79) flexible est fabriqué à base de nitinol.

10. Dispositif selon l'une quelconque des revendications 2 à 5, **caractérisé en ce que** la tige de couplage (19) et l'élément de couplage (46) sont reliés entre eux au moyen d'une articulation sphérique (50).

11. Dispositif selon l'une quelconque des revendications précédentes, **caractérisé en ce que** le dispositif de couplage (17) est conçu et/ou positionné dans l'état implanté de telle sorte que l'extrémité de couplage (18) touche la position de couplage (16) sans prétension statique.

12. Dispositif selon l'une quelconque des revendications 1 à 10, **caractérisé en ce que** le dispositif de couplage (17) est conçu et/ou est positionné dans l'état est implanté de telle sorte que l'extrémité de couplage (18) touche le point de couplage (16) avec une légère prétension.

13. Dispositif selon l'une quelconque des revendications précédentes, **caractérisé en ce qu'**un film d'humidité (44) est formé au moins dans l'état implanté entre l'extrémité de couplage (18) et le point de couplage (16).

14. Dispositif selon l'une quelconque des revendications précédentes, **caractérisé en ce que** l'extrémité de couplage (18) est conçue concave avant le couplage par rapport au point de couplage (16).

15. Dispositif selon l'une quelconque des revendications précédentes, **caractérisé en ce que** le point de couplage est formé par une plaquette de couplage (43) qui, dans l'état implanté, est reliée de façon fixe à la surface de la partie à contacter de la chaîne d'osselets, de la plaque de base à étrier ou d'une membrane terminant la fenêtre ronde ou une fenêtre artificielle.

16. Dispositif selon l'une quelconque des revendications précédentes, **caractérisé en ce que** l'élément de couplage (81, 88) a une structure de grande surface et percée sur l'extrémité de couplage.

17. Dispositif selon l'une quelconque des revendications 1 à 15, **caractérisé en ce que** l'élément de couplage (95) est muni sur l'extrémité de couplage de plusieurs bras (97) montés sur ressort.
